# EUROPEAN PATENT SPECIFICATION

(11) **EP 2 621 450 B1**
(45) Date of publication and mention of the grant of the patent: **23.03.2016**
(21) Application number: 11768223.7
(22) Date of filing: 26.09.2011
(51) Int. Cl.: A61J 1/20

(54) **TWO-PIECE VIAL TRANSFER NEEDLE ASSEMBLY**
ZWEITEILIGE AMPULLENTRANSFERNADELANORDNUNG
SYSTÈME D'AIGUILLE DE TRANSFERT DE FLACON À DEUX COMPOSANTS

(30) Priority: 28.09.2010 US 892073
(43) Date of publication of application: 07.08.2013
(73) Proprietor: Covidien LP, Mansfield, MA 02048 (US)
(72) Inventor: FINKE, Mel, DeLand FL 32720 (US); FOSTER, John, K., Port Orange FL 32128 (US)
(74) Representative: Gray, James
(86) International application number: PCT/US2011/053209
(87) International publication number: WO 2012/044565

(56) References cited:
- WO-A1-98/02129
- WO-A1-2011/039747
- DE-A1- 3 016 998
- US-A- 4 084 588
- US-A- 5 501 676
- US-A1- 2002 127 150
- US-B1- 6 478 788

## Description

### BACKGROUND

### Technical Field

The present disclosure relates to a vial transfer needle assembly and, more particularly, to a two-piece vial transfer needle assembly.

### Description of Related Art

In the medical field, certain types of solutions (e.g., drugs) are commonly stored in vials that utilize a pierceable cap (e.g., resealable septum) on one end and a plunger that is inserted into the opposite end. It is common for a clinician to use a vial transfer needle assembly that includes a spike or needle on one end and a standard threaded female luer connecter on the other end to fluidly couple the vial to a medical device, e.g., syringe, catheter, hubbed needle, etc.

Currently vial transfer needle assemblies arc manufactured by utilizing a three-piece design, which generally includes a plastic hub portion, a metal needle (e.g., a cannula), and a crimp-on metal insert that is used to capture the needle and seat it within the plastic hub portion. A sheath component may be pressed over the luer taper and serves as a sterile fluid path barrier when the hub is assembled to a vial.

US 6478788 B1 relates to packaging medicines and relates to a device for connection between a closed recipient and a container.

It would be beneficial to decrease the amount of parts needed for a vial transfer needle assembly and to simplify the assembly process.

### SUMMARY

The present disclosure relates to a vial transfer needle assembly including a first molding member and a second molding member. The first molding member includes a hub portion that has a proximal piercing tip and a distal portion. The hub portion defines a lumen that passes therethrough. The second molding member includes a central housing, a proximal skirt, and a distal skirt. The second molding portion is molded at least partially about the first molding portion to form a monolithic structure. The piercing tip is positioned to extend into a first cavity that is defined by the proximal skirt. The distal portion of the first molding member is positioned to extend through a second cavity that is defined by the distal skirt. The distal skirt is dimensioned to receive a medical device. The second molding member is configured to overlay and couple to the first molding member during an injection molding process.

In embodiments, the vial transfer needle assembly may include a removable sheath that is configured to cover the distal portion of the first molding member. The removable sheath may be molded with the second molding member. The removable sheath may include an annular wall that defines an open proximal portion and a closed distal portion. The removable sheath is configured to cover at least a portion of the first molding member.

In other embodiments, the closed distal portion of the removable sheath may include a textured configuration to facilitate handling by a user when the removable sheath is removed therefrom.

In embodiments, the open proximal end of the removable sheath may be formed on to a portion of the distal skirt via a frangible portion such that when the removable sheath is removed the frangible portion is configured to fracture to thereby facilitate removal of the removable sheath.

The first molding member includes a rigid structure having a higher Shore durometer than the second molding member. Whereas, the second molding member includes a flexible material having a lower Shore durometer than the first molding member to facilitate insertion of the vial when inserted into the proximal skirt.

In embodiments, the proximal skirt includes an inner wall and a base. The inner wall may include an annular bead that is disposed along an inner periphery of the inner wall. The annular bead is provided to facilitate releasable engagement of a vial when inserted within the first cavity of the proximal skirt.

In other embodiments, the distal skirt includes an inner wall and a base. The inner wall may have a plurality of threads disposed alongside the inner wall in a helical configuration for reception of the medical device, e.g., a catheter tube, within the second cavity of the distal skirt. Connecting structures other than helical threads are envisioned.

The vial transfer needle assembly may further include a cover that is disposed atop of an outer periphery of the proximal skirt to prevent contaminants from entering the cavity of the proximal skirt.

The present disclosure also relates to a method of manufacturing a vial transfer needle assembly. In an initial step, a first shot of a first material is injected in a form of a first molding member, the first molding member having a hub portion. Subsequently, a second shot of a second material having a lower Shore durometer than the first material is injected. The second shot is configured to at least partially overmold the first molding member. The second molding member includes a hub portion, a proximal skirt, a distal skirt, and a removable sheath. The second molding member overlays and couples to the first molding member.

### BRIEF DESCRIPTION OF THE DRAWINGS

Various embodiment of the subject instrument are described herein with reference to the drawings wherein:
Fig. 1 is a perspective view of a vial transfer needle assembly according to one embodiment of the present disclosure;
Fig. 2 is a side cross-sectional view of the vial transfer needle assembly of Fig. 1;
Fig. 3 is an enlarged view of the indicated area of detail of Fig. 2;
Fig. 4 is a side cross-sectional view of the vial transfer needle assembly shown in Fig. 1 with a vial having a plunger coupled thereto and a removable sheath shown removed therefrom;
Fig. 5 is an enlarged view of the indicated area of detail of Fig. 4;
Fig. 6A is a perspective view of the vial transfer needle assembly shown in Fig. 1 with a cover disposed over an opening of the vial transfer needle assembly;
Fig. 6B is a perspective view of the vial transfer needle assembly shown in Fig. 6A with the cover partially removed therefrom; and
Fig. 7 is a side cross-sectional view of the vial transfer needle assembly shown in Fig. 1 connected at one end to a vial and plunger assembly and a second end connected to a catheter tube.

### DETAILED DESCRIPTION

Embodiments of the presently disclosed vial transfer needle assembly are described in detail with reference to the drawings wherein like reference numerals identify similar or identical elements. As used herein, the term "distal" refers to that portion of the device which is further from a user while the term "proximal" refers to that portion of the device which is closer to a user.

The present invention is directed to a vial transfer needle. In order to release the solution from the vial, one end of the vial transfer needle assembly is snapped over the pierceable cap such that the spike of the vial transfer needle assembly pierces the septum of the cap. On the opposite end of the vial transfer needle assembly, a medical device is connected to the standard threaded female luer connection. The plunger in the vial is then pressed into the vial to transfer the contents of the vial through the vial transfer needle assembly and into the patient. Alternatively, the medical device, such as a syringe, connected to the threaded female luer connection may withdraw the contents from a vial through the vial transfer needle into the medical device for delivery to the patient. It is understood that the vial transfer needle of the present invention may also be used in conjunction with a plungerless vial, in which the transfer needle may also comprise self-venting features.

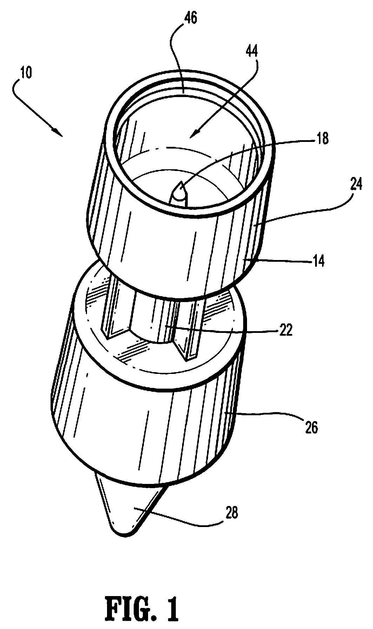

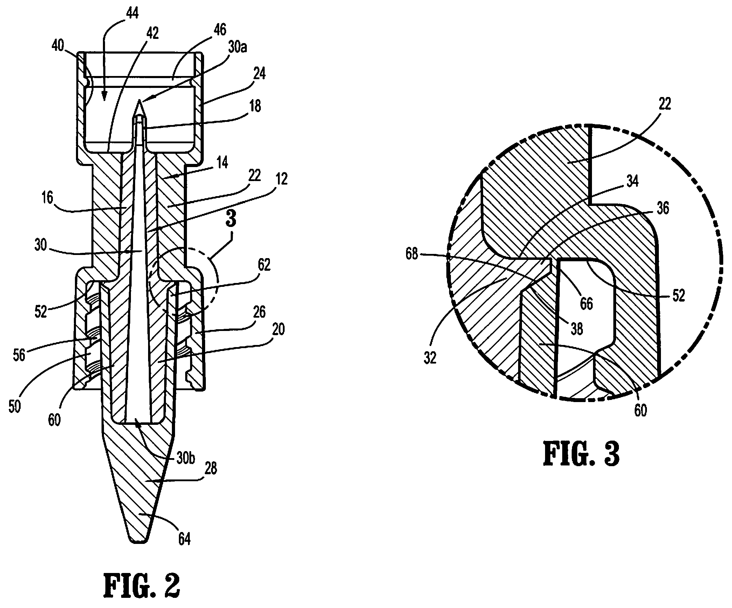

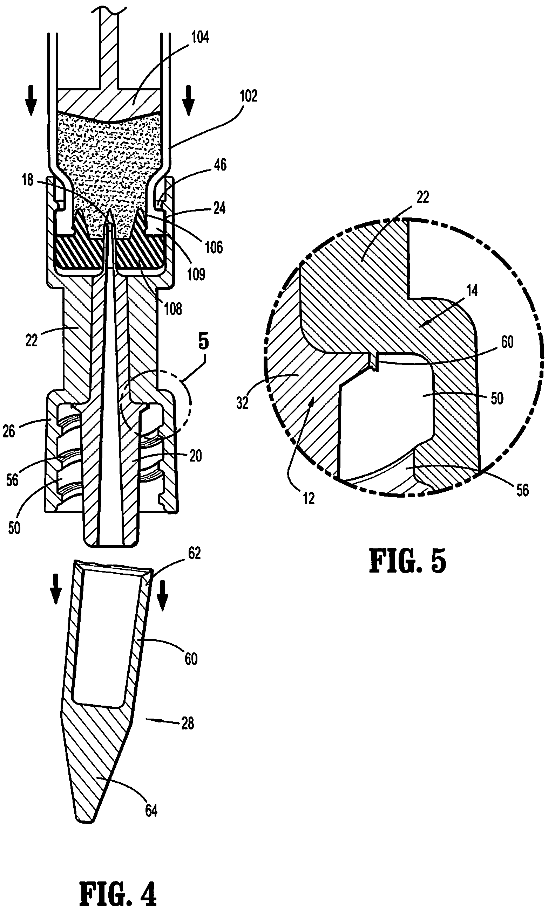

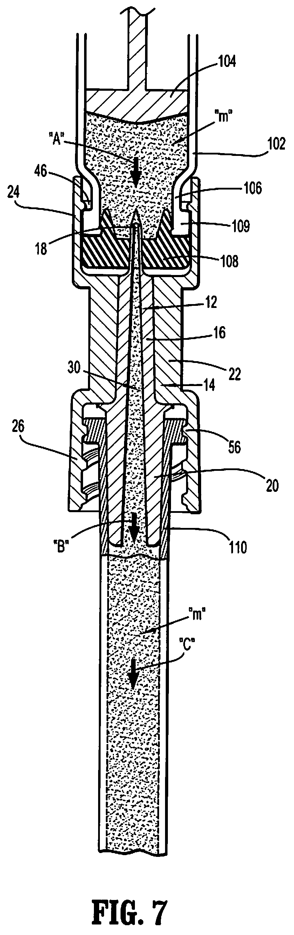

Referring initially to Figs. 1 and 2, a transfer needle assembly is shown and generally depicted as 10. Transfer needle assembly 10 is provided to transfer fluid from a vial 102 having a plunger 104 (Figs. 4 and 7) to a medical device. The medical device may be any medical device constructed and arranged to receive the contents of the vial and to releasably mate to the transfer needle assembly 10. For example, the medical device may be a needleless syringe or a catheter. In one embodiment shown in Fig. 7, the medical device is a catheter tube 110. Transfer needle assembly 10 generally includes a first molding member 12 and a second molding member 14 (Fig. 2) that is configured to overlay and couple to first molding member 12 during an injection molding process. In one embodiment, first molding member 12 is injected into a mold during a "first shot" of the injection molding process and, subsequently, second molding member 14 is injected over at least a portion of the first molding member 12 during a "second shot" of the injection molding process. In one embodiment, the first molding member 12 is entirely overmolded with second molding member 14. The two-shot molding process provides a benefit since it eliminates handling and costs associated with additional pieces and manufacturing steps.

Referring now to Fig. 2, first molding member 12 generally includes a central hub portion 16 having a proximal piercing tip 18 and a distal luer taper 20. Proximal piercing tip 18 is configured to pierce a pierceable septum of vial 102 (Figs. 4 and 7). Proximal piercing tip 18 may comprise any desired shape able to pierce the septum of the vial and to transfer the contents of the vial. The piercing tip 18 may be a blunt or sharp tipped. In one embodiment, the piercing tip 18 is constructed and arranged to minimize or prevent coring of the septum during piercing.

Distal luer taper 20 is configured to be received within the medical device such as a catheter tube 110 (shown in Fig. 7). In embodiments, first molding member 12 is made from a hard material (e.g., a polymer) to provide a rigid structure for the proximal piercing tip 18 and the distal luer taper 20. In embodiments, different colors may be used to distinguish first molding member 12 from second molding member 14. For example, first molding member 12 may include one colorant, while second molding member 14 may include a different colorant. In one embodiment, first molding member 12 having a particular piercing tip configuration may be associated with one color, while a first molding member having a different piercing tip configuration may be associated with a different color. In another embodiment, different colorants may be used to identify particular assembly features, such as gage and flow.

Referring also to Fig. 2, central hub portion 16 defines a lumen 30 having a proximal portion 30a and a distal portion 30b. Proximal portion 30a of lumen 30 extends to proximal piercing tip 18 and distal portion 30b of lumen 30 extends through distal luer taper 20. In embodiments, lumen 30 tapers from a larger diameter at distal portion 30b to a smaller diameter at proximal portion 30a (Fig. 2).

Central hub portion 16 also includes a shoulder 32 extending about its outer periphery to securely abut second molding member 14. Shoulder 32 includes a flat surface 34 (Fig. 3), a side surface 36 and an angled surface 38.

Second molding member 14 includes a central hub portion 22, a proximal skirt 24, a distal skirt 26 and a removable sheath 28. Proximal skirt 24 includes an inner wall 40 and a base 42 that define a cavity 44. Inner wall 40 includes an annular bead 46 that is disposed along an inner periphery of inner wall 40. Annular bead 46 is provided to facilitate releasable engagement of vial 102 within cavity 44 when the vial 102 is inserted into cavity 44 of proximal skirt 24. Other types of releasable engagement structures are known and envisioned for use in place of the annular bead. In embodiments, second molding member 14 is made from a softer material, for example, a polymer having a lower Shore durometer than first molding member 12, to allow for flexible manipulation of second molding member 14 during surgical use. For example, softer material enables proximal skirt 24 to expand when vial 102 is inserted therewithin.

Distal skirt 26 includes an inner wall 50 and a base 52 that together define a cavity 54. Inner wall 50 includes a plurality of threads 56 that are disposed alongside inner wall 50 in a helical fashion such that a catheter tube 110 (Fig. 7) or any other type of luer-type connecting conduit may be connected thereto. Connecting structure other than threads 56 is envisioned.

As discussed above, second molding member 14 includes a removable sheath 28 that is disposed within distal skirt 26. Removable sheath 28 includes an annular wall 60 defining an open proximal portion 62 and a closed distal portion 64. Removable sheath 28 is configured to cover distal luer taper 20 of first molding member 12. In embodiments, closed distal portion 64 of removable sheath 28 may have a textured and/or ergonomic configuration to facilitate handling by a user when being pulled and/or broken off second molding member 14.

Referring to Figs. 2 and 3, during the "second shot" of the injection molding process, open proximal end 62 of removable sheath 28 is releasably secured to base 52 of distal skirt 26 via a frangible portion 66 (e.g., very thin layer). Removable sheath 28 covers the open end of distal luer taper 20. As briefly discussed above, central hub portion 16 includes a shoulder 32, which abuts base 52 of distal skirt 26 to provide a firm placement of central hub portion 16 in relation to second molding member 14. In addition, during the "second shot" of the injection molding process, the softer material is configured to take a form over shoulder 32. More specifically, an angled surface 68 of proximal portion 62 of removable sheath 28 abuts angled surface 38 of shoulder 32, while frangible portion 66 surrounds side surface 36 of shoulder 32. Referring also to Figs. 4 and 5, frangible portion 66 is configured to easily break to facilitate removal of removable sheath 28 from second molding member 14 prior to use of vial transfer needle assembly 10.

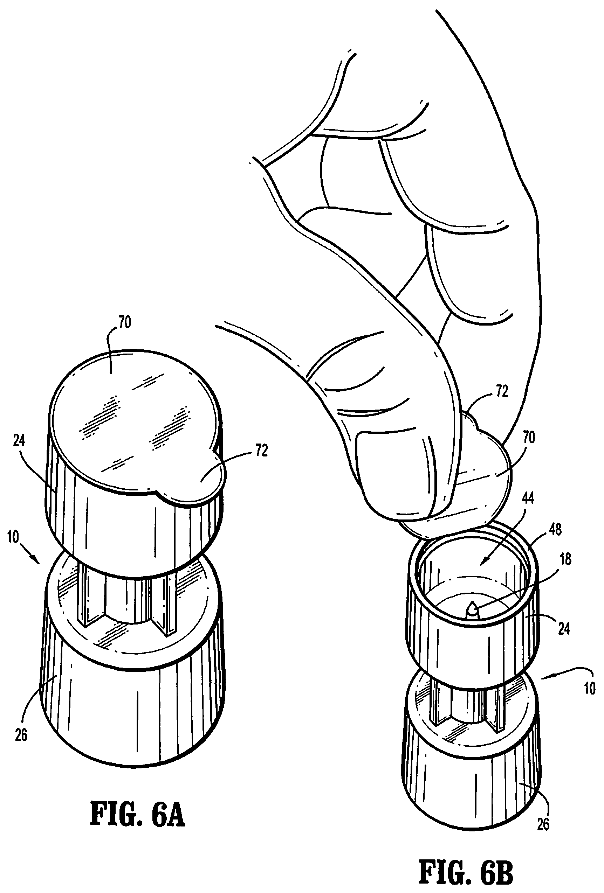

Referring to Figs. 6A and 6B, a cover 70 may be disposed over the opening defined by an outer periphery 48 of proximal skirt 24. Cover 70 may have a pull tab 72 and is configured to prevent any contaminants from entering cavity 44 of proximal skirt 24 during packaging and before a surgical procedure is performed. Cover 70 may be formed of any conventional material such as a coated paper, laminate or heat sealable film.

Referring to Fig. 7, as it is commonly known in the art, vial 102 may contain a medicinal fluid "M" or any other suitable types of fluids. As it is also commonly known in the art, vial 102 may include a plunger 104, a neck portion 106, and a pierceable septum 108 that is positioned within a rim 109 of vial 102.

During use, a clinician removes (e.g., peels off) cover 70 from outer periphery 48 of proximal skirt 24 and inserts vial 102 within cavity 44 of proximal skirt 24. As vial 102 is inserted within cavity 44, rim 109 of vial 102 is pushed passed annular bead 46 formed on an inner wall of proximal skirt 24 until septum 108 abuts base 42 of the proximal skirt 24. In this position, annular bead 46 is securely snug around neck portion 106, thus securing vial 102 within proximal skirt 24. Also in this position, proximal piercing tip 18 pierces the pierceable septum 108 of vial 102 to thereby allow medicinal fluid "M" to pass through proximal lumen 30a.

The clinician may then remove removable sheath 28 by twisting and/or pulling on removable sheath 28, thus breaking the frangible portion 66. After removing the removable sheath 28, the clinician then inserts a connecting structure, e.g., a luer connector, of the cannula tube 110 or other medical device, e.g., syringe, catheter, hubbed needle, etc., into distal skirt 26 into engagement with luer threads 56 such that distal luer taper 20 is disposed within cannula tube 110. Afterwards, plunger 104 of vial 102 is depressed such that the medicinal fluid "M" travels from vial 102 (as shown by arrow "A"), through central hub portion 16 and distal luer taper 20 (as shown by arrow "B") via lumen 30, and ultimately into catheter tube 110 (as shown by arrow "C").

It will be understood that various modifications may be made to the embodiments disclose herein. For example, the disclosed removable sheath may be provided after the disclosed second shot molding process. That is, the disclosed removable sheath may be made from a different material other than a polymer, such as, for example, any suitable type of metal. Therefore, the above description should not be construed as limiting, but merely as exemplifications of embodiments. Those skilled in the art will envision other modification within the scope and spirit of the claims appended hereto.

## Claims

1. A vial transfer needle assembly (10) comprising:
a first molding member (12) including a hub portion (16) having a proximal piercing tip (18) and a distal portion (30b), the hub portion (16) defining a lumen (30) which extends from the proximal piercing tip (18) through the distal portion (30b); and
a second molding member (14) including a central housing, a proximal skirt (24), and a distal skirt (26), the second molding portion being molded at least partially about the first molding portion to form a monolithic structure;
wherein the piercing tip (18) is positioned to extend into a first cavity (44) defined by the proximal skirt (24) and the distal portion (30b) of the first molding member (12) is positioned to extend through a second cavity (54) defined by the distal skirt (26), the distal skirt (26) dimensioned to receive a medical device, **characterized in that** the second molding member (14) is configured to overlay and couple to the first molding member (12) during an injection molding process.

2. The vial transfer needle assembly (10) according to Claim 1, further comprising a removable sheath (28) configured to cover the distal portion (30b) of the first molding member (12).

3. The vial transfer needle assembly (10) according to Claim 2, wherein the removable sheath (28) is molded integrally with the second molding member (14).

4. The vial transfer needle assembly (10) according to any one of claims 2-3, wherein the removable sheath (28) includes an annular wall (60) defining an open proximal portion (62) and a closed distal portion (64), the removable sheath (28) configured to cover at least a portion of the first molding member (12).

5. The vial transfer needle assembly (10) according to Claim 4, wherein the closed distal portion (64) of the removable sheath (28) includes a textured configuration to facilitate handling by a user when the removable sheath (28) is removed therefrom.

6. The vial transfer needle assembly (10) according to any one of claims 4-5, wherein the open proximal portion (62) of the removable sheath (28) is connected to a portion of the distal skirt (26) via a frangible portion (66) such that when the removable sheath (28) is removed from the distal skirt (26), the frangible portion (66) is configured to fracture.

7. The vial transfer needle assembly (10) according to any one of the preceding claims, wherein the first molding member (12) comprises a rigid structure having a higher Shore durometer than the second molding member (14).

8. The vial transfer needle assembly (10) according to any one of the preceding claims, wherein the lumen (30) of the hub portion (16) tapers from a larger diameter at the proximal portion (30a) to a smaller diameter at the distal portion (30b).

9. The vial transfer needle assembly (10) according to any one of the preceding claims, wherein the hub portion (16) includes a shoulder (32) extending about its outer periphery, the shoulder (32) having a flat surface (34), a side surface (36), and an angled surface (38).

10. The vial transfer needle assembly (10) according to any one of the preceding claims, wherein the proximal skirt (24) includes an inner wall (40) and a base (42), the inner wall (40) including an annular bead (46) that is disposed along an inner periphery of the inner wall (40), the annular bead (46) being provided to facilitate releasable engagement of a vial (102) within the first cavity (44) of the proximal skirt (24).

11. The vial transfer needle assembly (10) according to any one of the preceding claims, wherein the second molding member (14) comprises a flexible material having a lower Shore durometer than the first molding member (12) to facilitate insertion of the vial (102) into the proximal skirt (24).

12. The vial transfer needle assembly (10) according to any one of the preceding claims, wherein the distal skirt (26) includes an inner wall (50) and a base (52), the inner wall (50) having a plurality of threads (56) disposed alongside the inner wall (50) in a helical configuration.

13. The vial transfer needle assembly (10) according to any one of the preceding claims, further comprising a cover (70) for sealing a cavity (44) defined by the proximal skirt (24) to prevent contaminants from entering the cavity (44) of the proximal skirt (24).

14. A method of manufacturing a vial transfer needle assembly (10), the method comprising the steps of:
injection molding a first molding member (12), the first molding member (12) including a hub portion (16) having a proximal piercing tip (18) and a distal portion (30b), the hub portion (16) defining a lumen (30) extending from the proximal piercing tip (18) through the distal portion (30b); and
overmolding a second material having a lower Shore durometer than the first material about the first molding member (12) to define a second molding member (14) about the first molding member (12), the second molding member (14) having a hub portion (16), a proximal skirt (24) defining a first cavity (44), a distal skirt (26) defining a second cavity (54), and a removable sheath (28), and **characterized in that** the second molding member (14) overlays and couples to the first molding member (12).

## Patentansprüche

1. Ampullen-Transferkanülenanordnung (10), umfassend:
ein erstes Formteil (12) einschließlich eines Kanülenanschlussabschnitts (16) mit einer proximalen Durchdringungsspitze (18) und einem Endabschnitt (30b), wobei der Kanülenanschlussabschnitt (16) ein Lumen (30) definiert, das von der proximalen Durchdringungsspitze (18) durch den Endabschnitt (30b) verläuft; und
ein zweites Formteil (14) einschließlich eines zentralen Gehäuses, eines proximalen Rands (24) und eines Endrands (26), wobei das zweite Formteil zumindest teilweise um das erste Formteil geformt wird, um ein monolithisches Gefüge zu bilden;
wobei die Durchdringungsspitze (18) so positioniert ist, dass sie sich in einen ersten Hohlraum (44) erstreckt, der von dem proximalen Rand (24) definiert wird, und der Endabschnitt (30b) des ersten Formteils (12) so positioniert ist, dass er sich durch einen zweiten Hohlraum (54) erstreckt, der von dem Endrand (26) definiert wird, und der Endrand (26) so dimensioniert ist, dass er eine medizinische Vorrichtung aufnehmen kann, **dadurch gekennzeichnet, dass** das Formteil (14) dazu ausgestaltet ist, während eines Spritzgussverfahrens das erste Formteil (12) zu überdecken und sich damit zu verbinden.

2. Ampullen-Transferkanülenanordnung (10) nach Anspruch 1, weiter einen entfernbaren Schaft (28) umfassend, der dazu ausgestaltet ist, den Endabschnitt (30b) des ersten Formteils (12) abzudecken.

3. Ampullen-Transferkanülenanordnung (10) nach Anspruch 2, wobei der entfernbare Schaft (28) integral mit dem zweiten Formteil (14) geformt wird.

4. Ampullen-Transferkanülenanordnung (10) nach einem der Ansprüche 2-3, wobei der entfernbare Schaft (28) eine ringförmige Wand (60) umfasst, die einen offenem proximalen Abschnitt (62) und einen geschlossenen Endabschnitt (64) definiert, wobei der entfernbare Schaft (28) dazu ausgestaltet ist, mindestens einen Abschnitt des ersten Formteils (12) zu bedecken.

5. Ampullen-Transferkanülenanordnung (10) nach Anspruch 4, wobei der geschlossene Endabschnitt (64) des entfernbaren Schafts (28) eine texturierte Ausgestaltung umfasst, um die Handhabung durch einen Benutzer zu ermöglichen, wenn der entfernbare Schaft (28) davon entfernt wird.

6. Ampullen-Transferkanülenanordnung (10) nach einem der Ansprüche 4-5, wobei der offene proximale Abschnitt (62) des entfernbaren Schafts (28) mit einem Abschnitt des Endrands (26) über einen zerbrechlichen Abschnitt (66) verbunden ist, sodass bei Entfernen des entfernbaren Schafts (28) von dem Endrand (26), der zerbrechliche Abschnitt (66) dazu ausgestaltet ist, zu zerbrechen.

7. Ampullen-Transferkanülenanordnung (10) nach einem der vorangehenden Ansprüche, wobei das erste Formteil (12) ein starres Gefüge mit einem höheren Shore-Durometer als das zweite Formteil (14) umfasst.

8. Ampullen-Transferkanülenanordnung (10) nach einem der vorangehenden Ansprüche, wobei sich das Lumen (30) des Kanülenanschlussabschnitts (16) von einem größeren Durchmesser am proximalen Abschnitt (30a) zu einem kleineren Durchmesser am Endabschnitt (30b) verjüngt.

9. Ampullen-Transferkanülenanordnung (10) nach einem der vorangehenden Ansprüche, wobei der Kanülenanschlussabschnitt (16) eine Schulter (32) umfasst, die sich in seine äußere Peripherie erstreckt, wobei die Schulter (32) eine flache Oberfläche (34), eine Seitenfläche (36) und eine gewinkelte Fläche (38) aufweist.

10. Ampullen-Transferkanülenanordnung (10) nach einem der vorangehenden Ansprüche, wobei der proximale Rand (24) eine Innenwand (40) und eine Basis (42) umfasst, wobei die Innenwand (40) einen ringförmigen Wulst (46) umfasst, der sich entlang einer inneren Peripherie der Innenwand (40) befindet, und der ringförmige Wulst (46) dazu bereitgestellt wird, um ein wieder lösbares Eingreifen einer Ampulle (102) in den ersten Hohlraum (44) des proximalen Rands (24) zu ermöglichen.

11. Ampullen-Transferkanülenanordnung (10) nach einem der vorangehenden Ansprüche, wobei das zweite Formteil (14) ein flexibles Material umfasst, das einen niedrigeren Shore-Durometer aufweist als das erste Formteil (12), um die Einführung der Ampulle (102) in den proximalen Rand (24) zu ermöglichen.

12. Ampullen-Transferkanülenanordnung (10) nach einem der vorangehenden Ansprüche, wobei der Endrand (26) eine Innenwand (50) und eine Basis (52) umfasst, wobei die innere Wand (50) eine Vielzahl von Gewinden (56) umfasst, die entlang der Innenwand (50) spiralförmig positioniert sind.

13. Ampullen-Transferkanülenanordnung (10) nach einem der vorangehenden Ansprüche, weiter eine Abdeckung (70) zur Abdichtung eines Hohlraums (44) umfassend, der von dem proximalen Rand (24) definiert wird, um Schmutzstoffe davon abzuhalten, in den Hohlraum (44) des proximalen Rands (24) einzudringen.

14. Verfahren zum Herstellen einer Ampullen-Transferkanülenanordnung (10), wobei das Verfahren die folgenden Schritte umfasst:
Spritzgießen eines ersten Formteils (12), wobei das erste Formteil (12) einen Kanülenanschlussabschnitt (16) mit einer proximalen Durchdringungsspitze (18) und einem Endabschnitt (30b) beinhaltet, wobei der Kanülenanschlussabschnitt (16) ein Lumen (30) definiert, das von der proximalen Durchdringungsspitze (18) durch den Endabschnitt (30b) verläuft; und
Überspritzen eines zweiten Materials, das einen niedrigeren Shore-Durometer aufweist als das erste Material, um das erste Formteil (12), um ein zweites Formteil (14) um das erste Formteil (12) zu definieren, wobei das zweite Formteil (14) einen Kanülenanschlussabschnitt (16), einen proximalen Rand (24), der einen ersten Hohlraum (44) definiert, einen Endrand (26), der einen zweiten Hohlraum (54) definiert, und einen entfernbaren Schaft (28) aufweist, und **dadurch gekennzeichnet, dass** das zweite Formteil (14) das erste Formteil (12) überdeckt und sich damit verbindet.

## Revendications

1. Ensemble à aiguille de transfert vers une fiole (10) comprenant :
un premier élément de moulage (12) contenant une partie formant un moyeu (16) comportant un embout de perçage proximal (18) et une partie distale (30b), la partie formant un moyeu (16) définissant un lumen (30) qui s'étend à partir de l'embout de perçage proximal (18) à travers la partie distale (30b) ; et
un deuxième élément de moulage (14) contenant un boîtier central, une collerette proximale (24) et une collerette distale (26), la deuxième partie de moulage étant moulée au moins en partie autour de la première partie de moulage pour former une structure monolithique ;
l'embout de perçage (18) étant positionné pour s'étendre dans une première cavité (44) définie par la collerette proximale (24) et la partie distale (30b) du premier élément de moulage (12) étant positionnée pour s'étendre à travers une deuxième cavité (54) définie par la collerette distale (26), la collerette distale (26) étant dimensionnée pour recevoir un dispositif médical, **caractérisé en ce que** le deuxième élément de moulage (14) est conçu pour recouvrir et pour se coupler au premier élément de moulage (12) lors d'un processus de moulage par injection.

2. Ensemble à aiguille de transfert vers une fiole (10) selon la revendication 1, comprenant en outre une gaine amovible (28) conçue pour couvrir la partie distale (30b) du premier élément de moulage (12).

3. Ensemble à aiguille de transfert vers une fiole (10) selon la revendication 2, dont la gaine amovible (28) est moulée intégralement avec le deuxième élément de moulage (14).

4. Ensemble à aiguille de transfert vers une fiole (10) selon l'une quelconque des revendications 2 ou 3, dont la gaine amovible (28) comprend une paroi annulaire (60) définissant une partie proximale ouverte (62) et une partie distale fermée (64), la gaine amovible (28) étant conçue pour couvrir au moins une partie du premier élément de moulage (12).

5. Ensemble à aiguille de transfert vers une fiole (10) selon la revendication 4, dont la partie distale fermée (64) de la gaine amovible (28) présente une configuration texturée pour faciliter la manipulation par un utilisateur lorsque la gaine amovible (28) est retirée de l'ensemble.

6. Ensemble à aiguille de transfert vers une fiole (10) selon l'une quelconque des revendications 4 ou 5, dont la partie proximale ouverte (62) de la gaine amovible (28) est raccordée à une partie de la collerette distale (26) par le biais d'une partie fragile (66) de sorte que quand la gaine amovible (28) est enlevée de la collerette distale (26), la partie fragile (66) est conçue pour rompre.

7. Ensemble à aiguille de transfert vers une fiole (10) selon l'une quelconque des revendications précédentes, dont le premier élément de moulage (12) comprend une structure rigide présentant une dureté Shore supérieure à celle du deuxième élément de moulage (14).

8. Ensemble à aiguille de transfert vers une fiole (10) selon l'une quelconque des revendications précédentes, dont le lumen (30) de la partie formant un moyeu (16) se réduit d'un grand diamètre au niveau de la partie proximale (30a) à un plus petit diamètre à la partie distale (30b).

9. Ensemble à aiguille de transfert vers une fiole (10) selon l'une quelconque des revendications précédentes, dont la partie formant un moyeu (16) comprend un épaulement (32) s'étendant autour de sa périphérie extérieure, l'épaulement (32) présentant une surface plane (34), une surface latérale (36) et une surface en biais (38).

10. Ensemble à aiguille de transfert vers une fiole (10) selon l'une quelconque des revendications précédentes, dont la collerette proximale (24) comprend une paroi intérieure (40) et une base (42), la paroi intérieure (40) comprenant une perle annulaire (46) qui est disposée le long d'une périphérie intérieure de la paroi intérieure (40), la perle annulaire (46) étant conçue pour faciliter un engagement libérable d'une fiole (102) dans la première cavité (44) de la collerette proximale (24).

11. Ensemble à aiguille de transfert vers une fiole (10) selon l'une quelconque des revendications précédentes, dont le deuxième élément de moulage (14) comprend un matériau flexible dont la dureté Shore est inférieure à celle du premier élément de moulage (12) pour faciliter l'insertion de la fiole (102) dans la collerette proximale (24).

12. Ensemble à aiguille de transfert vers une fiole (10) selon l'une quelconque des revendications précédentes, dont la collerette distale (26) comprend une paroi intérieure (50) et une base (52), la paroi intérieure (50) comportant une pluralité de filets (56) disposés le long de la paroi intérieure (50) selon une configuration hélicoïdale.

13. Ensemble à aiguille de transfert vers une fiole (10) selon l'une quelconque des revendications précédentes, comprenant en outre un couvercle (70) servant à sceller une cavité (44) définie par la collerette proximale (24) pour empêcher que des contaminants de pénètrent dans la cavité (44) de la collerette proximale (24).

14. Procédé de fabrication d'un ensemble à aiguille de transfert vers une fiole (10), le procédé comprenant les étapes suivantes :
le moulage par injection d'un premier élément de moulage (12), le premier élément de moulage (12) comprenant une partie formant un moyeu (16) comportant un embout de perçage proximal (18) et une partie distale (30b), la partie formant un moyeu (16) définissant un lumen (30) s'étendant à partir de l'embout de perçage proximal à travers la partie distale (30b) ; et
le surmoulage d'un deuxième matériau dont la dureté Shore est inférieure à celle du premier matériau autour du premier élément de moulage (12) pour définir un deuxième élément de moulage (14) autour du premier élément de moulage (12), le deuxième élément de moulage (14) comportant une partie formant un moyeu (16), une collerette proximale (24) définissant une première cavité (44), une collerette distale (26) définissant une deuxième cavité (54) et une gaine amovible (28), et **caractérisé en ce que** le deuxième élément de moulage (14) recouvre le premier élément de moulage (12) se couple avec celui-ci.
